# EUROPEAN PATENT SPECIFICATION

(11) **EP 2 478 856 B1**
(45) Date of publication and mention of the grant of the patent: **12.10.2016**
(21) Application number: 12151485.5
(22) Date of filing: 18.01.2012
(51) Int. Cl.: A61B 17/34, A61B 10/02

(54) **Treatment device**
Behandlungsvorrichtung
Dispositif de traitement

(30) Priority: 24.01.2011 KR 20110006976; 24.01.2011 KR 20110006978
(43) Date of publication of application: 25.07.2012
(73) Proprietor: Samsung Medison Co., Ltd., Nam-myun Hongchun-gun Kangwon do 250-875 (KR)
(72) Inventor: Hyoun, Dong Gyu, Gyeonggi-do (KR); Song, Young Seuk, Seoul (KR)
(74) Representative: Schmid, Wolfgang

(56) References cited:
- EP-A2- 0 102 800
- DE-A1- 10 058 370
- US-A- 6 146 329
- US-A1- 2007 016 067

## Description

### BACKGROUND

### 1. Field

Embodiments of the present invention relate to a treatment device to measure a movement distance of a needle, thereby improving stability of a biopsy.

### 2. Description of the Related Art

A needle biopsy is used as a method of diagnosing a target suspected to be a tumor and choosing a treatment method based on such diagnosis.

An image guided needle biopsy using ultrasonic waves, an X-ray, magnetic resonance imaging (MRI) or computer tomography (CT) is used for needle biopsy, in which a needle is inserted into a human body, which is invisible.

A needle guide to guide the needle is provided in a probe, which is an ultrasonic diagnostic device. The needle moves along the needle guide to sample a target.

The above-mentioned technology is merely a related art provided to assist in understanding the prevent invention, which is not known in the art to which the present invention pertains.

In an ultrasonic image, the needle, moving to the front of the probe, is not easily distinguished from tissue around the needle. If a needle insertion route is not properly displayed in an image projected on an imaging device, it may be difficult for a user to confirm the movement distance of the needle with the result that a medical malpractice possibility may be increased.

A treatment device according to the preamble of claim 1 is known from US patent no. 6 146 329.

### SUMMARY

It is an aspect of the present invention to provide a treatment device that accurately measures a movement distance of a needle, thereby improving stability of a biopsy. Additional aspects of the invention will be set forth in part in the description which follows and, in part, will be obvious from the description, or may be learned by practice of the invention.

In accordance with one aspect of the present invention, a treatment device according to claim 1 is provided.

### BRIEF DESCRIPTION OF THE DRAWINGS

The aspects of the invention will become apparent and more readily appreciated from the following description of the embodiments, taken in conjunction with reference to the accompanying drawings of which:
FIG. 1 is a perspective view showing a main unit connected to a treatment device according to an embodiment of the present invention;
FIG. 2 is an exploded perspective view of the treatment device according to the embodiment of the present invention;
FIG. 3 is an assembled perspective view of the treatment device according to the embodiment of the present invention;
FIG. 4 is a perspective view, partially cutaway, showing the construction of a measurement unit according to an embodiment of the present invention;
FIG. 5 is a sectional view showing a state of a needle of the treatment device before being withdrawn from a needle guide according to the embodiment of the present invention;
FIG. 6 is a sectional view showing engagement between a linear gear and a circular gear measured by a camera according to an embodiment of the present invention;
FIG. 7 is a sectional view showing symbols to calculate a length of the needle of the treatment device inserted into a target according to the embodiment of the present invention;
FIG. 8 is a block diagram of the treatment device according to the embodiment of the present invention;
FIG. 9 is a sectional view showing a state of a needle of a treatment device before moving along a needle guide according to another embodiment of the present invention;
FIG. 10 is a sectional view showing a state in which the needle shown in FIG. 9 moves along the needle guide;
FIG. 11 is a sectional view showing a state of a needle of a treatment device before being withdrawn from a needle guide according to another embodiment of the present invention;
FIG. 12 is a sectional view showing a state in which a pressure protrusion is sensed while passing a pressure sensor according to another embodiment of the present invention;
FIG. 13 is a perspective view showing a needle including a pressure sensor and a pressure protrusion according to another embodiment of the present invention;
FIG. 14 is an exploded perspective view showing a treatment device according to another embodiment of the present invention;
FIG. 15 is an assembled perspective view of the treatment device according to an embodiment of the present invention;
FIG. 16 is a sectional view showing a state of a needle of the treatment device before entering an inlet according to an embodiment of the present invention; and
FIG. 17 is a sectional view showing a state in which the needle of the treatment device has reached a reference position after entering the inlet according to an embodiment of the present invention.

### DETAILED DESCRIPTION

Reference will now be made in detail to embodiments of the present invention, examples of which are illustrated in the accompanying drawings, wherein like reference numerals refer to like elements throughout. For convenience of description, a medical treatment device will be described as an example. In the drawings, the thickness of each line or size of each component may be exaggerated for convenience of description and clarity. Also, terms, which will be described below, may be defined considering functions of the present invention, and may vary according to usual practice or the intention of users or operators. Consequently, such terms may be defined based on the specification.

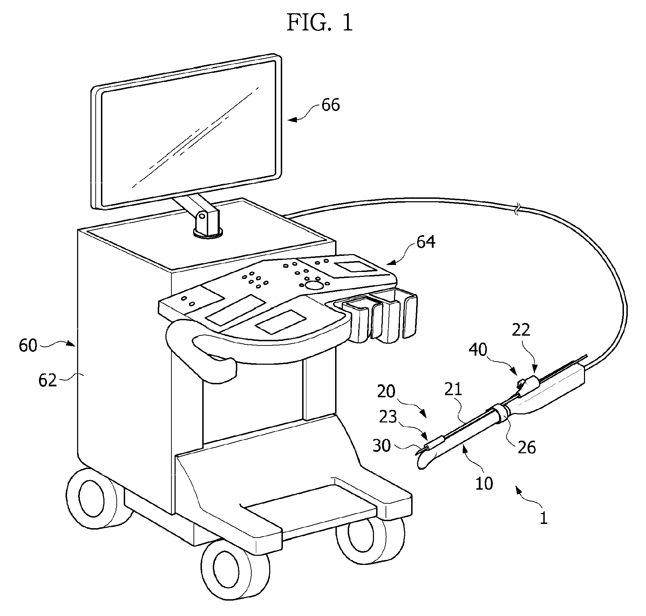

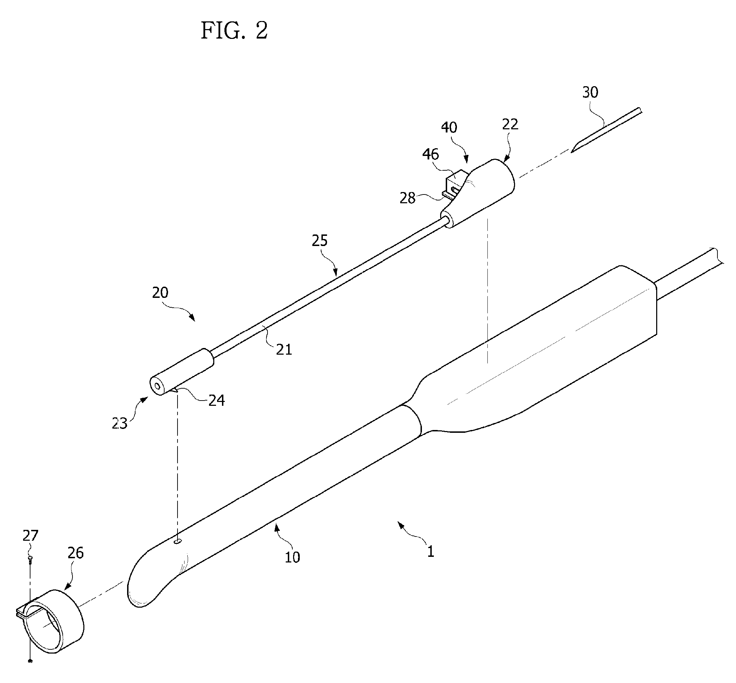

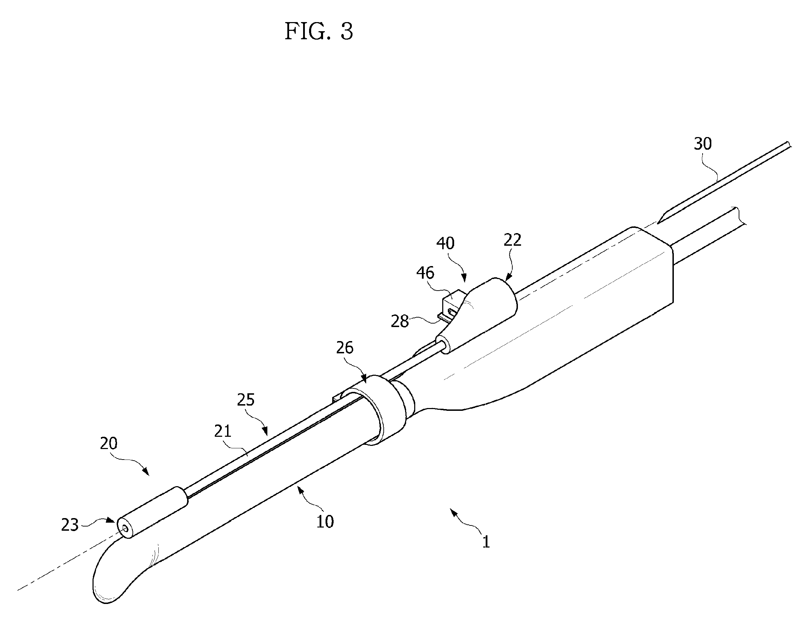

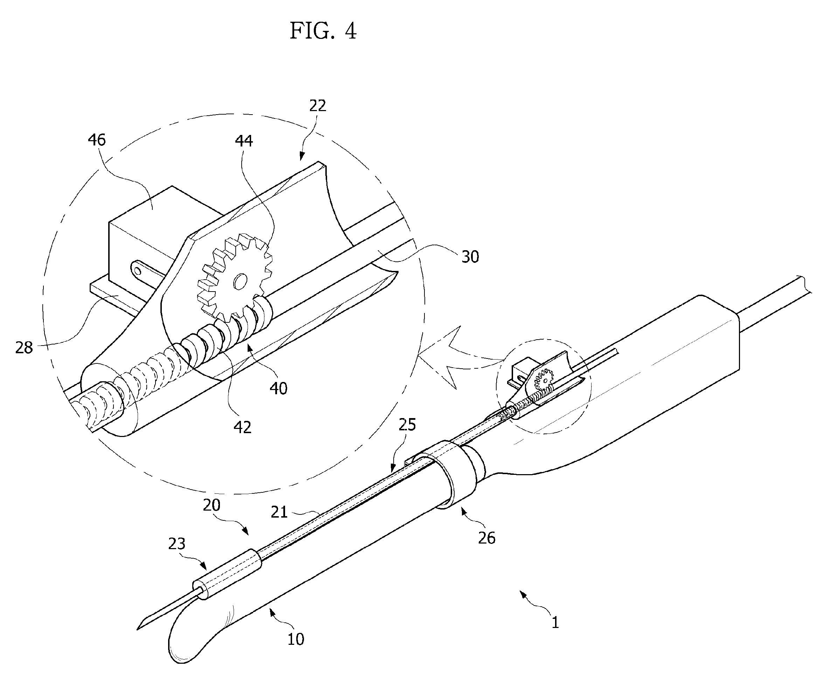

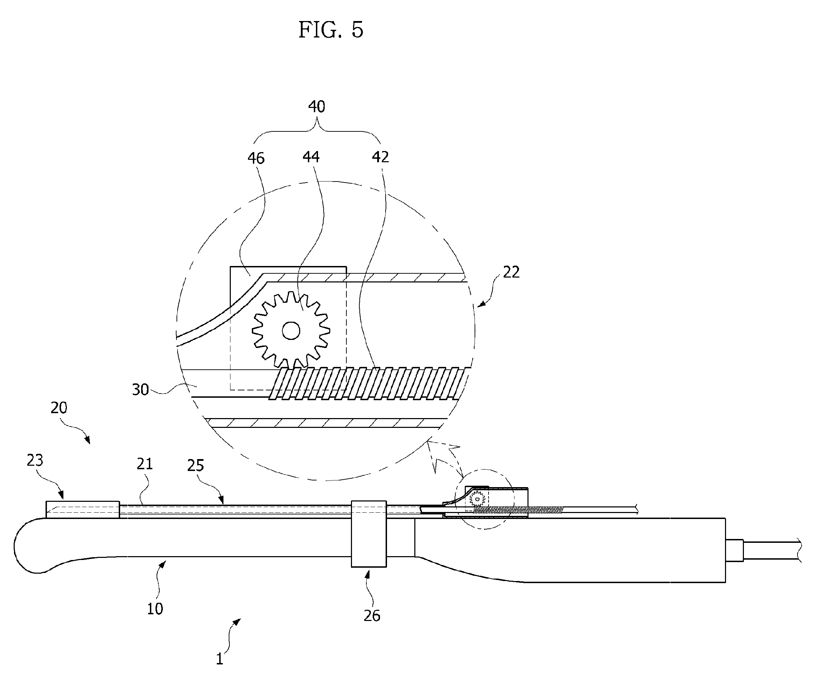

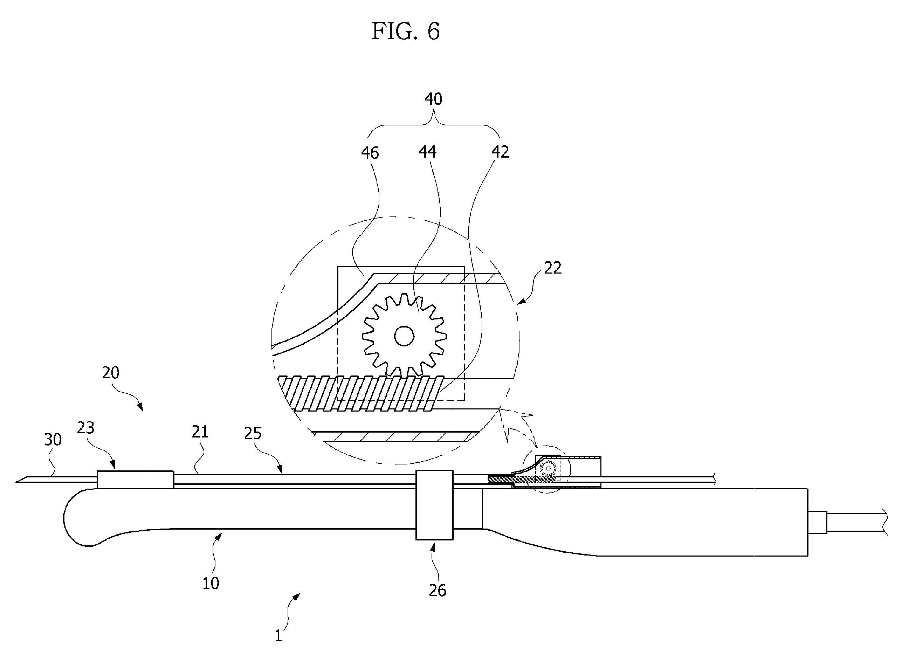

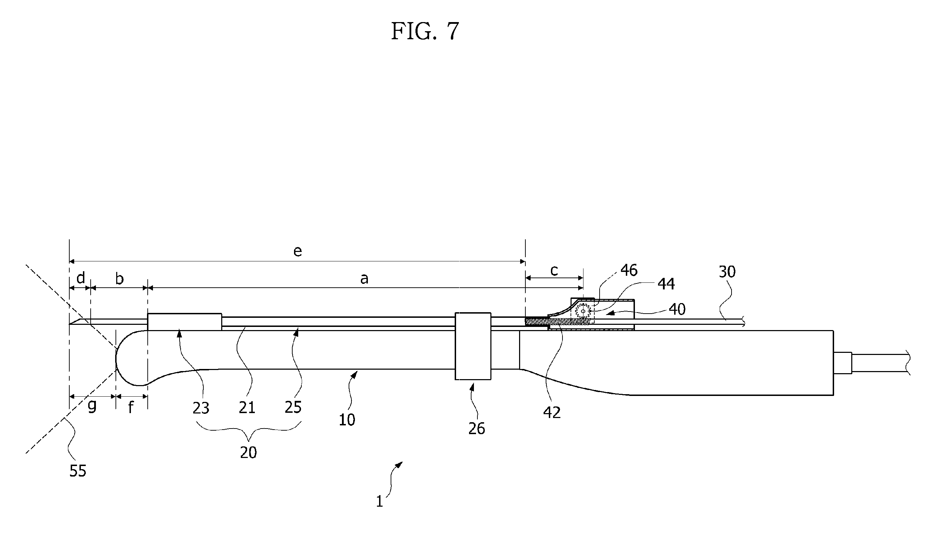

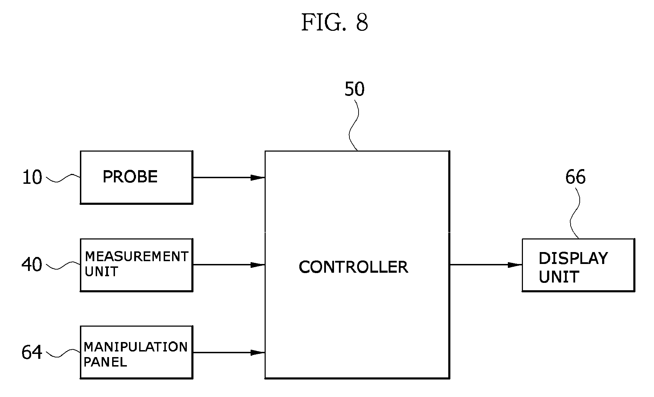

FIG. 1 is a perspective view showing a main unit connected to a treatment device according to an embodiment of the present invention, FIG. 2 is an exploded perspective view of the treatment device according to the embodiment of the present invention, FIG. 3 is an assembled perspective view of the treatment device according to the embodiment of the present invention, FIG. 4 is a perspective view, partially cutaway, showing the construction of a measurement unit according to an embodiment of the present invention, FIG. 5 is a sectional view showing a state of a needle of the treatment device before being withdrawn from a needle guide according to the embodiment of the present invention, FIG. 6 is a sectional view showing engagement between a linear gear and a circular gear measured by a camera according to an embodiment of the present invention, FIG. 7 is a sectional view showing symbols to calculate a length of the needle of the treatment device inserted into a target according to the embodiment of the present invention, and FIG. 8 is a block diagram of the treatment device according to the embodiment of the present invention.

As shown in FIGS. 1 to 8, a treatment device 1 includes a probe 10 to acquire an image of a target (not shown) using ultrasonic waves, a needle guide 20 provided along the probe 10, a needle 30 movable along the needle guide 20, a measurement unit 40 to sense a movement distance of the needle 30 based on contact caused by movement of the needle 30, and a controller 50 to calculate the movement distance of the needle 30 based on a value measured by the measurement unit 40.

Various ultrasonic diagnostic devices may be used as the probe 10 as long as the ultrasonic diagnostic devices acquire an image of a target using ultrasonic waves.

In this embodiment, the probe 10 transmits an ultrasonic signal to a target and receives an ultrasonic signal reflected from the target to acquire an ultrasonic image of the target.

The probe 10 is connected to a main unit 60 so that the ultrasonic signal received by the probe 10 is transmitted to the main unit 60.

The main unit 60 includes a main body 62, in which various kinds of equipment are installed, a manipulation panel 64 connected to the main body 62 to allow a user to input a manipulation signal, and a display unit 66 to display the signal received by the probe 10.

The needle guide 20 is provided along the probe 10. The needle guide 20 may be formed in various shapes as long as the needle guide 20 guides movement of the needle 30.

In this embodiment, the needle guide 20 includes a guide pipe 21 provided along the probe 10 to define a passage therein, an inlet 22 provided at one side (right side in FIG. 2) of the guide pipe 21, and an outlet 23 provided at the other side (left side in FIG. 2) of the guide pipe 21.

The outlet 23 is a portion which is inserted into a human body, and therefore, the measurement unit 40, which senses the movement distance of the needle 30, is disposed in the inlet 22 or the middle 25 of the needle guide 20 so that the outlet 23 is sanitarily maintained.

The inlet 22 has a diameter greater than that of the guide pipe 21 so that the needle 30 easily enters the guide pipe 21. A portion of the inlet 22 connected to the guide pipe 21 is inclined to guide movement of the needle 30.

A hook 24 protruding from the outlet 23 is inserted in an opening of the probe 10 to fix the outlet 23 to the probe 10.

A connection member 26 connected to the inlet 22 or the guide pipe 21 is formed in the shape of a band. In this embodiment, the connection member 26 is provided to wrap the probe 10 and the guide pipe 21 of the needle guide 20. Opposite ends of the connection member 26 are fixed by a fastening bolt 27.

The needle 30 moves along the guide pipe 21 through the inlet 22 and is withdrawn from the needle guide 20 through the outlet 23 so that the needle 30 samples or treats a target.

The needle 30 may be formed in various shapes as long as the needle 30 moves along the needle guide 20 to sample or treat the target.

The movement distance of the needle 30 withdrawn from the needle guide 20 is measured by the measurement unit 40 and is transmitted to the controller 50.

The needle 30 may include an insertion part which is inserted into a target, e.g., a human body, to be measured, and an installation part at which the measurement unit 40 is installed, to avoid infection during measurement.

That is, the insertion part and the installation part of the needle 30 are separated from each other so that the installation part is disinfected or replaced, thereby preventing infection during measurement.

The measurement unit 40 may be variously modified as long as the measurement unit 40 senses a movement distance of the needle 30 based on contact caused by movement of the needle 30.

In this embodiment, the measurement unit 40 includes a linear gear 42 formed along the needle 30, a circular gear 44 configured to rotate in engagement with the linear gear 42, and a rotation measurement member 46 to measure rotation of the circular gear 44.

The circular gear 44 engages with the linear gear 42 immediately before the needle 30 is withdrawn from the guide pipe 21 through the outlet 23. The circular gear 44 is rotated in proportion to a distance in which the needle 30 moves outward through the outlet 23.

The gear formation section and the gear shape of the linear gear 42 may be modified as long as the linear gear 42 is formed at the outside of the needle 30 so as to engage with the circular gear 44.

The linear gear 42 may protrude from the outside of the needle 30. Alternatively, the linear gear 42 may be formed at the inside of the needle 30 in the shape of a groove. Also, the linear gear 42 is formed at the outside of the needle 30 in proportion to the distance of the needle 30 moving outward from the needle guide 20 through the outlet 23.

The circular gear 44, rotating while engaging with the linear gear 42, is disposed inside the inlet 22 of the needle guide 20. The rotation measurement member 46, axially connected to the circular gear 44, is disposed outside of the inlet 22.

The rotation measurement member 46 is fixed by a support 28 extending from the inlet 22. The rotation measurement member 46 may be disposed in the needle guide 20. As needed, the rotation measurement member 46 may be fixedly disposed in the probe 10.

Also, in this embodiment, the rotation measurement member 46 and the circular gear 44 may be disposed at the inlet 22 of the needle guide 20. As needed, the rotation measurement member 46 and the circular gear 44 may be disposed at the middle 25 of the needle guide 20.

In a case in which the measurement unit 40 is provided in the inlet 22, germs are prevented from moving along the needle 30 to provide more sanitary test environments since external contamination sources from the front end (left side in FIG. 6) of the needle 30 are relatively distant from the inlet 22.

In a case in which the inlet 22 has too small a space to receive the measurement unit 40, the measurement unit 40 may be provided in the middle 25 of the needle guide 20 between the inlet 22 and the outlet 23 to improve space utilization.

Since the construction of the measurement unit 40 to measure a movement distance of the needle 30 is relatively simple, the measurement unit 40 may weigh 1 kg or less. Consequently, a user may easily manipulate the treatment device 1 including the measurement unit 40.

Various sensors may be used in the rotation measurement member 46 used in the measurement unit 40 as long as the sensors measure the number of rotations of the circular gear 44 and transmit a measured value to the controller 50.

In this embodiment, a rotary encoder is used as the rotation measurement member 46 to measure rotation of the circular gear 44.

As shown in FIG. 8, the controller 50 according to this embodiment calculates a movement distance of the needle 30 based on a value measured by the measurement unit 40, and an ultrasonic image signal measured by the probe 10 is also transmitted to the controller 50.

Also, the manipulation panel 64, which allows a user to input a manipulation signal, is connected to the controller 50 to transmit a signal to the controller 50. The controller 50 synthesizes and outputs signals from the probe 10, the measurement unit 40 and the manipulation panel 64 to the display unit 66.

The controller 50 may be disposed at one or more selected from the probe 10, the needle guide 20 and the main unit 60.

Hereinafter, the operation of the treatment device 1 according to this embodiment will be described in detail with reference to the accompanying drawings.

As shown in FIG. 3, the needle 30 is moved toward the inlet 22 of the needle guide 20. Subsequently, as shown in FIG. 5, the needle 30 is inserted through the inlet 22 and is moved along the guide pipe 21.

In a state in which the front end of the needle 30 is placed in the outlet 23 of the needle guide 20, the circular gear 44, which is disposed in the inlet 22, engages with the linear gear 42.

As the needle 30 is withdrawn through the outlet 23, as shown in FIGS. 4 and 6, the linear gear 42 moves along with the needle 30 with the result that the circular gear 44 is rotated.

The rotation of the circular gear 44 is measured by the rotation measurement member 46. A value measured by the rotation measurement member 46 is transmitted to the controller 50.

The controller 50 calculates the movement distance of the needle 30 based on the value measured by the rotation measurement member 46 and provides the result to a user.

The controller 50 may provide the movement distance of the needle 30 to the user through the display unit 66. Alternatively, an additional speaker may be used to provide the movement distance of the needle 30 to the user.

The user easily confirms the movement distance of the needle 30 through the display unit 66, thereby more safely sampling a target.

Various calculation expressions and methods may be used to calculate the length of the needle 30 inserted into a human body using the treatment device 1 according to this embodiment.

In this embodiment, as shown in FIG. 7, the distance from the front end (left side in FIG. 7) of the outlet 23 to the rotation measurement member 46 is expressed by a symbol 'a'.

A beam irradiation surface 55, to which ultrasonic waves are irradiated to acquire an image, is provided at the front (left side in FIG. 7) of the probe 10. The distance from an interface between the beam irradiation surface 55 and the needle 30 to the front end of the outlet 23 is expressed by a symbol 'b'.

The distance from a zero position (left side in FIG. 7), which is a reference position of the linear gear 42, to the rotation measurement member 46 of the measurement unit 40 to sense a position is expressed by a symbol 'c'. A value of 'c' may be acquired through a measurement by the measurement unit 40.

The distance from the zero position, which is the reference position of the linear gear 42, to the front end (left side in FIG. 7) of the needle 30 is expressed by a symbol 'e'.

Values of 'a', 'b' and 'e' may be kinematically calculated or acquired through real measurement. These values are stored in the controller 50.

The value of 'c' may be acquired through operation of the measurement unit 40 according to movement of the needle 30. This value is transmitted to the controller 50.

The distance d from the interface between the beam irradiation surface 55 and the needle 30 to the front end (left side in FIG. 7) of the needle 30 may be acquired based on these values.

That is, a symbol 'd' is a length of needle 30 displayed only on the beam irradiation surface 55. A value of 'd' may be acquired using the following expression: 'd=(c+e)-(a+b)'.

The front end (left side in FIG. 7) of the probe 10 is a part contacting a target to be tested, i.e., a human body. The distance from the front end of the probe 10 to the front end of the needle 30 is a length of the needle 30 inserted into the human body, which is expressed by a symbol 'g'.

Also, the length from the front end of the probe 10 to the front end of the outlet 23 is expressed by a symbol 'f'. A value of 'f' may be kinematically calculated or acquired through real measurement.

Consequently, the length g of the needle inserted into the human body is calculated using the following expression: 'g=(b+d)-f'.

Values of 'b' and 'f' are stored in the controller 50. The expression to calculate the value of 'd' is substituted into the expression to calculate the value of 'g' to derive the following expression: 'g=(c+e)-(a+f)'.

These calculation expressions and methods may be used in an electronic measurement unit using an optical sensor as well as mechanical measurement units 40, 70 and 80 to measure the movement distance of the needle 30.

Hereinafter, a treatment device 2 according to another embodiment of the present invention will be described with reference to the accompanying drawings.

For convenience of description, components of this embodiment identical in construction and operation to those of the previous embodiment shown in FIGS. 1 to 8 are denoted by the same reference numerals, and a detailed description thereof will be omitted.

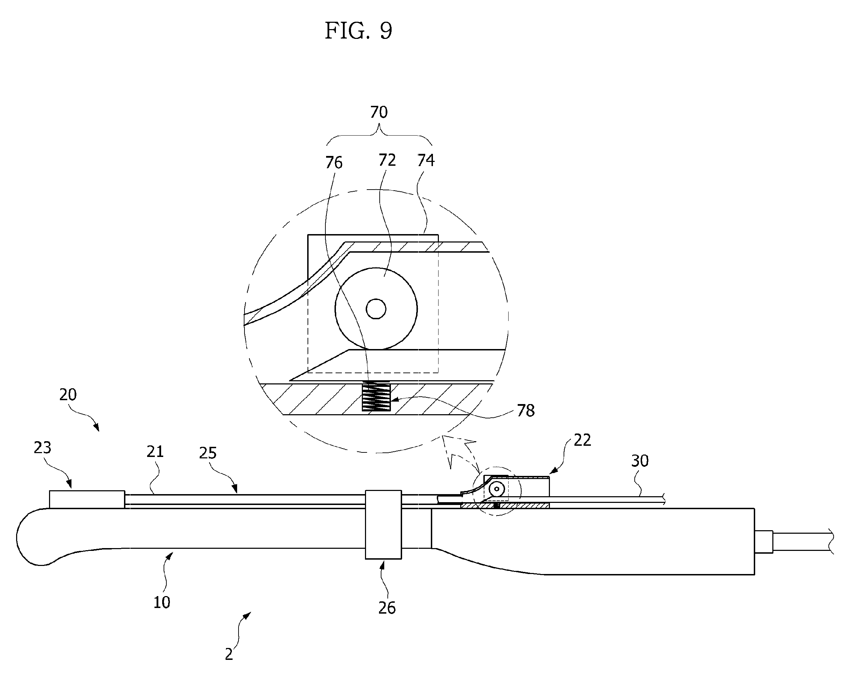

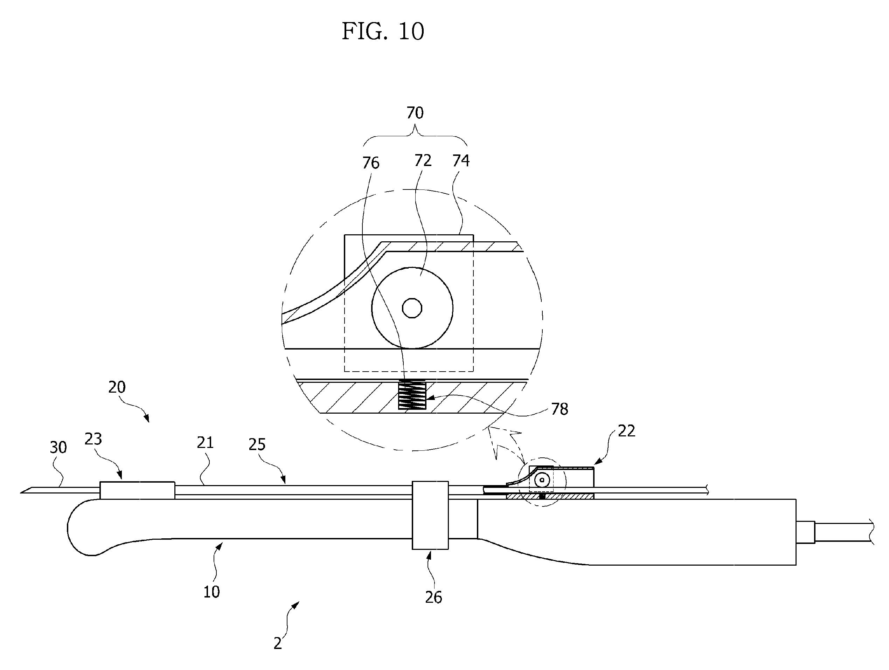

FIG. 9 is a sectional view showing a state of a needle of a treatment device before moving along a needle guide according to another embodiment of the present invention, and FIG. 10 is a sectional view showing a state in which the needle shown in FIG. 9 moves along the needle guide.

As shown in FIGS. 9 and 10, a measurement unit 70 of the treatment device 2 includes a roller 72 configured to rotate in contact with the needle 30, a rotation measurement member 74 to measure rotation of the roller 72, and an elastic pressing member 76 to elastically press the needle 30 toward the roller 72.

The roller 72 is rotatably disposed inside the inlet 22. The roller 72 may be formed of various materials and in various shapes as long as the roller 72 rotates in contact with the side of the needle 30.

In this embodiment, the roller 72 is mainly formed of rubber exhibiting a high coefficient of friction and elasticity.

The roller 72 may be provided in the needle guide 20 or the probe 10 so that the roller 72 rotates in contact with the side of the needle 30.

The elastic pressing member 76 is provided at the lower part of the inlet 22 inside thereof to improve contact force between the side of the needle 30 and the roller 72.

Various elastic pressing devices may be used as the elastic pressing member 76 as long as the elastic pressing devices press the needle 30 to the roller 72.

In this embodiment, a coil spring, which exhibits a high performance to price ratio, is used as the elastic pressing member 76.

The elastic pressing member 76 is disposed in an installation groove 78 formed inside the inlet 22 to press the needle 30 toward the roller 72.

Hereinafter, the operation of the treatment device 2 according to this embodiment will be described in detail with reference to the accompanying drawings.

As shown in FIG. 9, the needle 30 is inserted into the needle guide 20 through the inlet 22 and moved between the roller 72 and the elastic pressing member 76.

Since the needle 30 is moved in a state in which the side of the needle 30 is in contact with the roller 72, the roller 72 is rotated.

The rotation measurement member 74, connected to the roller 72, measures rotation of the roller 72. A value measured by the rotation measurement member 74 is transmitted to the controller 50.

The controller 50 calculates the movement distance of the needle based on the value measured by the rotation measurement member 74 and provides the result to a user.

The controller 50 calculates the movement distance of the needle by multiplying the radius of the roller 72 by an accumulated rotation angle of the roller 72.

The radius of the roller 72 may be kinematically calculated or acquired through real measurement. The radius of the roller 72 is stored in the controller 50.

Rotation angles of the roller 72, which is rotated in contact with the needle 30, are sensed by the rotation measurement member 74 and transmitted to the controller 50. The controller 50 sums the rotation angles of the roller 72 to acquire an accumulated rotation angle of the roller 72.

The controller 50 calculates the movement distance of the needle by multiplying the radius of the roller 72 by the accumulated rotation angle of the roller 72 and provides the result to the user through the display unit 66.

As the rotation measurement member 46 used in the previous embodiment and the rotation measurement member 74 used in this embodiment, an optical sensor may be used to measure the rotation angle in addition to the rotary encoders.

In a case in which the optical sensor is used as the rotation measurement member 6 or the rotation measurement member 74, a slit may be formed in the circular gear 44 or the roller 72, and a device to transmit and receive light in a direction in which the slit is rotated may be disposed to measure rotation angles and the number of rotations.

In addition, various optical sensors to measure rotation angles and the number of rotations using light may be used as the rotation measurement member.

Hereinafter, a treatment device 3 according to another embodiment of the present invention will be described with reference to the accompanying drawings.

For convenience of description, components of this embodiment identical in construction and operation to those of the previous embodiment shown in FIGS. 1 to 8 are denoted by the same reference numerals, and a detailed description thereof will be omitted.

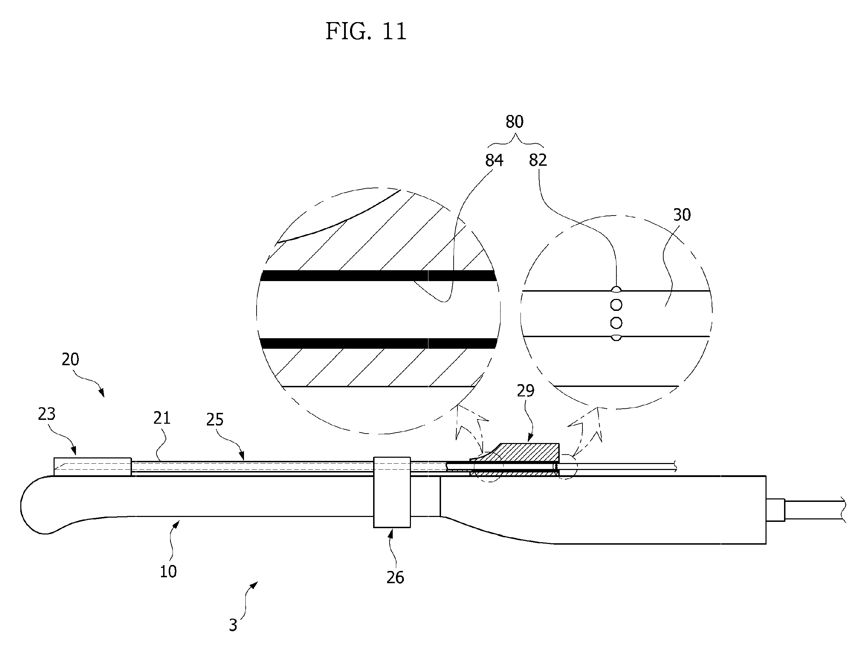

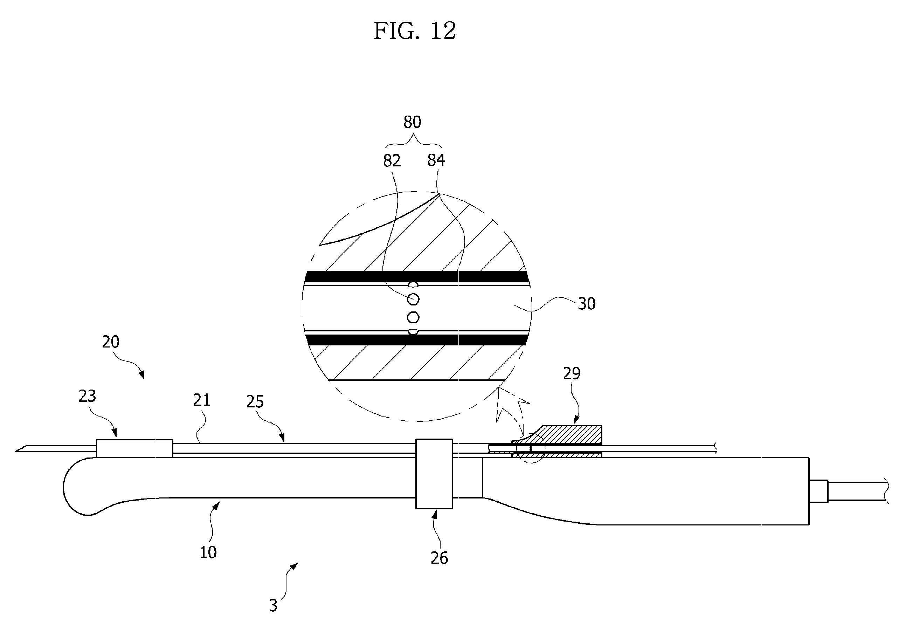

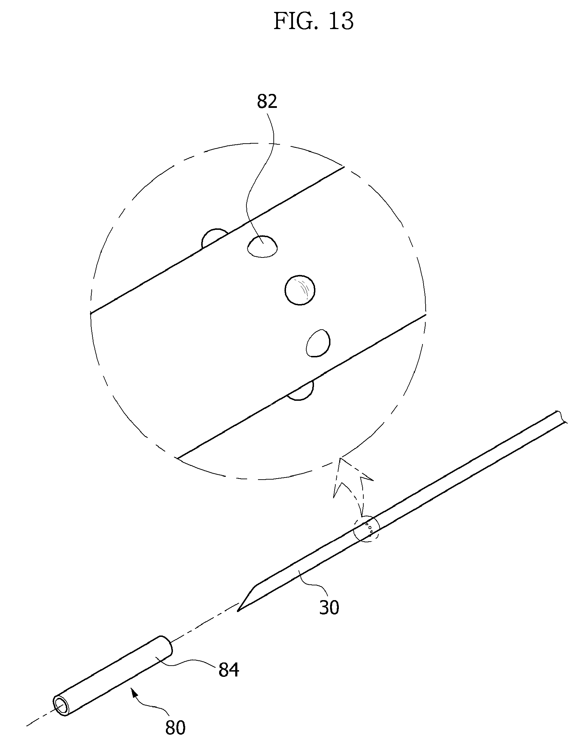

FIG. 11 is a sectional view showing a state of a needle of a treatment device before being withdrawn from a needle guide according to another embodiment of the present invention, FIG. 12 is a sectional view showing a state in which a pressure protrusion is sensed while passing a pressure sensor according to another embodiment of the present invention, and FIG. 13 is a perspective view showing a needle including a pressure sensor and a pressure protrusion according to another embodiment of the present invention.

As shown in FIGS. 11 to 13, a measurement unit 80 of the treatment device 3 includes a pressure protrusion 82 formed on the needle 30 in a protruding fashion and a pressure sensor 84 in contact with the pressure protrusion 82 to measure the position of the pressure protrusion 82.

The pressure protrusion 82 may be formed in various shapes as long as the pressure sensor 84 contacts the pressure protrusion 82 to measure the position of the pressure protrusion 82.

In this embodiment, the pressure protrusion 82 includes a plurality of protruding portions formed along the circumference of the needle 30.

Since the protruding portions of the pressure protrusion 82 are disposed along the circumference of the needle 30, the pressure protrusion 82 is easily measured, irrespective of the position at which the needle 30 is moved, thereby improving operational reliability.

The pressure sensor 84, which measures movement of the pressure protrusion 82, is formed in the shape of a cylinder surrounding the outside of the needle 30, thereby more stably measuring the pressure protrusion 82.

A value measured by the pressure sensor 84 is changed depending upon the change in position of the pressure protrusion 82, which moves along with needle 30. The controller 50 calculates the movement distance of the needle 30 based on the value measured by the pressure sensor 84 and provides the result to a user. Hereinafter, the operation of the treatment device 3 according to this embodiment will be described in detail with reference to the accompanying drawings.

As shown in FIG. 11, the front end of the needle 30 is moved along the guide pipe 21 though the inlet 29 and is placed in the outlet 23. The pressure protrusion 82 protruding outward from the needle 30 is located inside the pressure sensor 84.

As the needle 30 is moved outward through the outlet 23, as shown in FIG. 12, the pressure protrusion 82 moves along with the needle 30 with the result that a value measured by the pressure sensor 84 is changed.

The value measured by the pressure sensor 84 is transmitted to the controller 50. The controller 50 calculates the movement distance of the needle 30 based on the value measured by the pressure sensor 84 and provides the result to a user.

As described above, the treatment device 1, 2 or 3 senses the movement distance of the needle 30 based on contact caused by movement of the needle 30. Consequently, the movement distance of the needle 30 is accurately measured, thereby improving the stability of a biopsy.

Also, the measurement unit 40, 70 or 80 is provided with the needle 30 in the needle guide 20. Consequently, no additional space is needed, thereby improving space utilization.

A treatment device according to an embodiment of the present invention may further include a needle sensing unit 120 to determine whether the needle 30 has reached a reference position 110. The measurement unit 40 is driven based on the determination of the needle sensing unit 120 to measure the movement distance of the needle 30.

The reference position means a position at which the measurement unit begins to measure the movement distance of the needle 30 when the needle 30 reaches the position.

Hereinafter, the treatment device further including the needle sensing unit 120 will be described in detail.

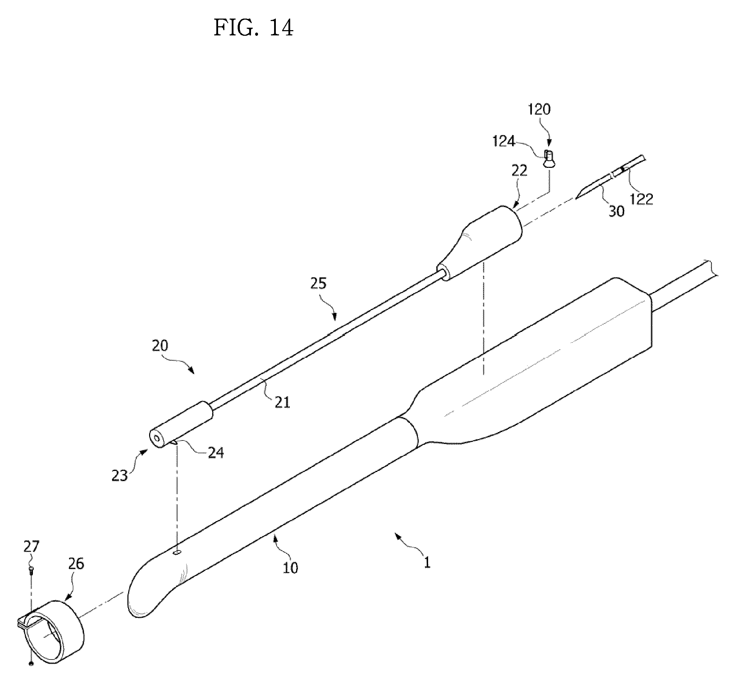

As shown in FIGS. 14 to 17, the treatment device includes a probe 10 to acquire an image of a target using ultrasonic waves, a needle guide 20 provided along the probe 10, a needle 30 movable along the needle guide 20, a needle sensing unit 120 to determine whether the needle 30 has reached a reference position, a measurement unit 40 driven based on the determination of the needle sensing unit 120 to measure a movement distance of the needle 30, and a controller 50 to calculate the movement distance of the needle 30 based on a value measured by the measurement unit 40.

An inlet 22 may have a diameter greater than that of a guide pipe 21 over a longer section than the previous embodiments so that the needle sensing unit 120 is disposed in the inlet 22.

The treatment device according to this embodiment may be constituted by further including the needle sensing unit 120 in the treatment device according to any one of the previous embodiments. Hereinafter, a treatment device constituted by further including the needle sensing unit 120 in the treatment device according to the previous embodiment shown in FIGS. 1 to 8 will be described for convenience of description.

FIG. 14 is an exploded perspective view showing a treatment device according to another embodiment of the present invention. Components of this embodiment identical in construction and operation to those of the previous embodiment shown in FIGS. 1 to 8 will not be described.

A contact type sensor or a noncontact type sensor may be used as the needle sensing unit 120, which drives the measurement unit 40.

Referring to FIG. 14, the needle sensing unit 120 includes a recognition member 122 provided on the outside of the needle 30 and a recognition sensor 124 to recognize the recognition member 122 to drive the needle sensing unit 120.

The recognition member 122 may be formed in various shapes as long as the recognition member 122 is recognized by the recognition sensor 124.

In this embodiment, the recognition member 122 is disposed on the outside of the needle 30 in the shape of a band. Consequently, the recognition member 122 is easily recognized by the recognition sensor 124 irrespective of directivity of the needle 30.

The recognition sensor 124 is operated in a contact or noncontact fashion to recognize the recognition member 122. In this embodiment, the recognition sensor 124 recognizes the recognition member 122 through transmission and reception of light. Consequently, operational reliability of the recognition sensor 124 is secured although the recognition sensor 124 is repeatedly used.

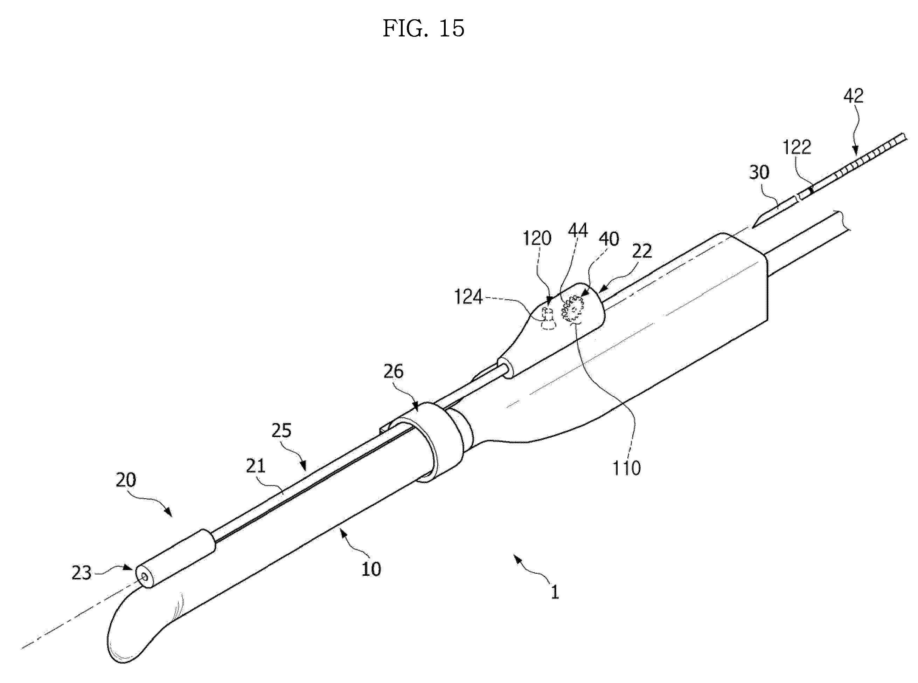

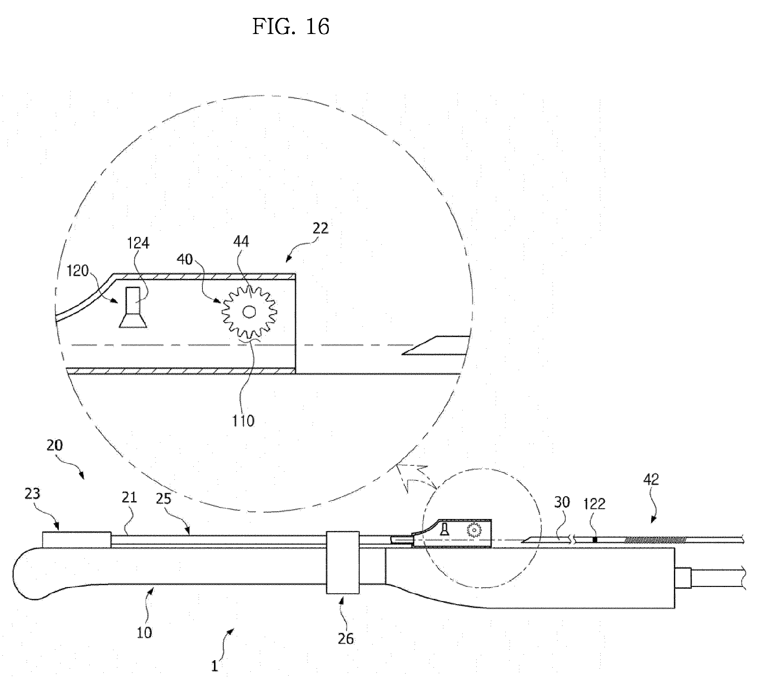

FIG. 15 is an assembled perspective view of the treatment device according to an embodiment of the present invention, and FIG. 16 is a sectional view showing a state of a needle of the treatment device before entering an inlet according to an embodiment of the present invention.

Referring to FIGS. 15 and 16, the needle 30 is moved toward the inlet 22 of the needle guide 20. Subsequently, the needle 30 is inserted through the inlet 22 and is moved along the guide pipe 21.

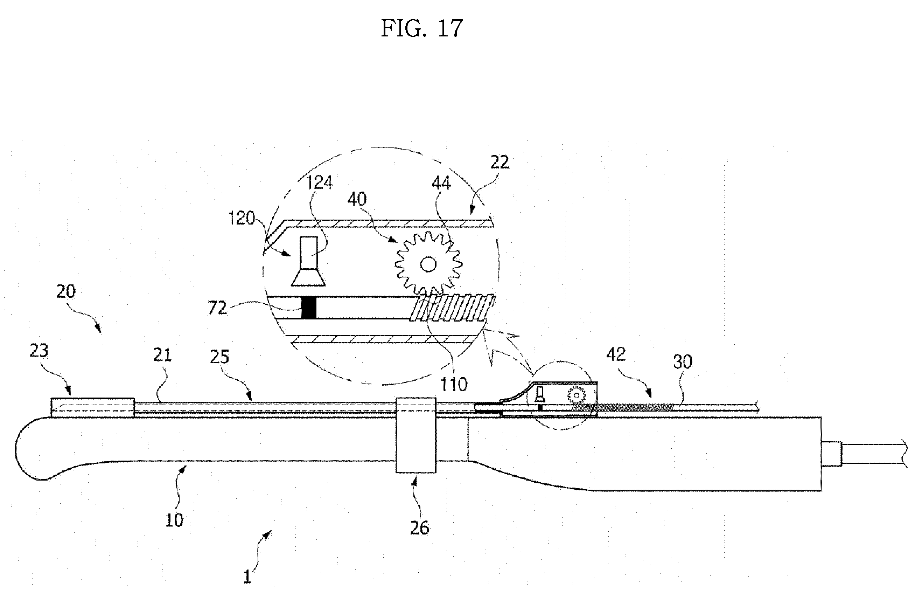

Referring to FIG. 17, when the recognition member 122 is recognized by the recognition sensor 124 in a state in which the front end of the needle 30 is placed in the outlet 23 of the needle guide 20, the needle sensing unit 120 transmits a signal to the controller 50, which transmits a drive signal to the measurement unit 40.

Alternatively, the recognition sensor 124 may directly transmit a drive signal to the measurement unit 40 so that the rotation of the circular gear 44 is measured by the rotation measurement member 46.

In the treatment device with the above-stated construction, the measurement unit 40 measures length information of the needle 30 based on the operation of the needle sensing unit 120 to determine whether the needle 30 has reached the reference position 110. Consequently, contamination of the needle 30 is prevented, and the movement distance of the needle 30 is accurately measured, thereby improving the stability of a biopsy.

Although this embodiment is constituted by including the needle sensing unit 120 in the previous embodiment shown in FIGS. 1 to 8, the needle sensing unit 120 may be included in the previous embodiment shown in FIGS. 9 and 10 or in the previous embodiment shown in FIGS. 11 to 13. Also, the treatment device to measure the movement distance of the needle based on contact caused by movement of the needle is not limited to the above embodiments. Consequently, the treatment device including the needle sensing unit 120 may further include an operation sensor in addition to the measurement units according to the previous embodiments shown in FIGS. 1 to 13 within a range in which a person having ordinary skill in the art may modify the treatment device.

In the above, medical treatment devices were described; however, embodiments of the present invention may be applied to other fields.

As is apparent from the above description, in the treatment device according to the embodiments of the present invention, the movement distance of the needle is sensed based on contact caused by movement of the needle, and the measurement unit measures length information of the needle based on the operation of the needle sensing unit to determine whether the needle has reached the reference position. Consequently, the movement distance of the needle is accurately measured, thereby improving the stability of a biopsy.

Also, the measurement unit is provided with the needle in the needle guide. Consequently, no additional space is needed, thereby improving space utilization.

## Claims

1. A treatment device comprising:
a probe (10) to acquire an image of a target;
a needle guide (20) provided along the probe (10);
a needle (30) movable along the needle guide (20);
**characterized in that** the treatment device further comprises:
a needle sensing unit (120) to determine whether the needle (30) has reached a reference position;
a measurement unit (40, 70, 80) driven based on a determination of the needle sensing unit (120), wherein the measurement unit (40, 70, 80) is to sense a movement distance of the needle (30) from the reference position based on contact between the measurement unit (40, 70, 80) and the needle (30) moving along the needle guide (20); and
a controller (50) to calculate the movement distance of the needle (30) based on a value measured by the measurement unit (40, 70, 80).

2. The treatment device according to claim 1, wherein the measurement unit (40) comprises:
a linear gear (42) formed along the needle (30);
a circular gear (44) configured to rotate in engagement with the linear gear (42); and
a rotation measurement member (46) to measure rotation of the circular gear (44).

3. The treatment device according to claim 1, wherein the measurement unit (70) comprises:
a roller (72) configured to rotate in contact with the needle (30); and
a rotation measurement member (46) to measure rotation of the roller (72).

4. The treatment device according to claim 2 or 3, wherein the rotation measurement member (46) is provided at the probe (10) or the needle guide (20).

5. The treatment device according to claim 2 or 3, wherein the rotation measurement member (46) is disposed in a middle or in an inlet of the needle guide (20).

6. The treatment device according to claim 3, wherein the measurement unit (70) further comprises an elastic pressing member (74) to elastically press the needle (30) toward the roller (72).

7. The treatment device according to claim 1, wherein the measurement unit (80) comprises:
a pressure protrusion (82) formed on the needle (30) in a protruding fashion;
and
a pressure sensor (84) in contact with the pressure protrusion (82) to measure a position of the pressure protrusion (82).

8. The treatment device according to claim 7, wherein the pressure protrusion (82) is formed along a circumference of the needle (30).

9. The treatment device according to claim 7, wherein the pressure sensor (84) is formed in a cylindrical shape surrounding an outside of the needle (30).

10. The treatment device according to claim 1, wherein the controller (50) is disposed at any one or more selected from the probe (10), the needle guide (20) and a main unit (60).

11. The treatment device according to claim 1, wherein the needle sensing unit (120) comprises:
a recognition member (122) provided on an outside of the needle (30); and
a recognition sensor (124) to recognize the recognition member (122) to drive the measurement unit (40, 70, 80).

12. The treatment device according to claim 11, wherein the recognition member (122) is disposed on the outside of the needle (30) in the shape of a band.

13. The treatment device according to claim 11, wherein the recognition sensor (124) recognizes the recognition member (122) through transmission and reception of light.

## Patentansprüche

1. Behandlungsvorrichtung, welche Folgendes aufweist:
eine Sonde (10), um ein Bild eines Ziels zu erlangen;
eine Nadelführung (20), die entlang der Sonde (10) vorgesehen ist;
eine Nadel (30), die entlang der Nadelführung (20) bewegbar ist;
**dadurch gekennzeichnet, dass**
die Behandlungsvorrichtung des Weiteren Folgendes aufweist:
eine Nadelerkennungseinheit (120), um zu ermitteln, ob die Nadel (30) eine Referenzposition erreicht hat;
eine Messeinheit (40,70,80), die von einer Ermittlung der Nadelermittlungseinheit (120) basierend angetrieben ist, wobei die Messeinheit (40,70,80) dafür vorgesehen ist, eine Bewegungsdistanz der Nadel (30) von der Referenzposition basierend auf einem Kontakt zwischen der Messeinheit (40,70,80) und der sich entlang der Nadelführung (20) bewegenden Nadel (30) zu detektieren; und
ein Steuergerät (50), um die Bewegungsdistanz der Nadel (30) basierend auf einem von der Messeinheit (40,70,80) gemessenen Wert zu berechnen.

2. Behandlungsvorrichtung nach Anspruch 1, wobei die Messeinheit (40) Folgendes aufweist:
ein lineares Zahnrad (42), das entlang der Nadel (30) gebildet ist;
ein kreisförmiges Zahnrad (44), das dafür vorgesehen ist, in Eingriff mit dem linearen Zahnrad (42) zu rotieren; und
ein Rotationsmesselement (46), um die Rotation des kreisförmigen Zahnrads (44) zu messen.

3. Behandlungsvorrichtung nach Anspruch 1, wobei die Messeinheit (70) Folgendes aufweist:
eine Rolle (72), die dafür vorgesehen ist, in Kontakt mit der Nadel (30) zu rotieren; und
ein Rotationsmesselement (46), um die Rotation der Rolle (72) zu messen.

4. Behandlungsvorrichtung nach Anspruch 2 oder 3, wobei das Rotationsmesselement (46) an der Sonde (10) oder der Nadelführung (20) vorgesehen ist.

5. Behandlungsvorrichtung nach Anspruch 2 oder 3, wobei das Rotationsmesselement (46) in einer Mitte oder in einem Einlass der Nadelführung (20) angeordnet ist.

6. Behandlungsvorrichtung nach Anspruch 3, wobei die Messeinheit (70) des Weiteren ein elastisches Presselement (74) aufweist, um die Nadel (30) elastisch in Richtung der Rolle (72) zu pressen.

7. Behandlungsvorrichtung nach Anspruch 1, wobei die Messeinheit (80) Folgendes aufweist:
einen Druckvorsprung (82), der auf der Nadel (30) in einer überstehenden Art und Weise gebildet ist; und
einen Drucksensor (84) in Kontakt mit dem Druckvorsprung (82), um eine Position des Druckvorsprungs (82) zu messen.

8. Behandlungsvorrichtung nach Anspruch 7, wobei der Druckvorsprung (82) entlang eines Umfangs der Nadel (30) gebildet ist.

9. Behandlungsvorrichtung nach Anspruch 7, wobei der Drucksensor (84) in einer zylindrischen Form gebildet ist, die eine Außenseite der Nadel (30) umgibt.

10. Behandlungsvorrichtung nach Anspruch 1, wobei das Steuergerät (50) an einem oder mehreren angeordnet ist, die aus der Sonde (10), der Nadelführung (20) und einer Haupteinheit (60) ausgewählt sind.

11. Behandlungsvorrichtung nach Anspruch 1, wobei die Nadelermittlungseinheit (120) Folgendes aufweist:
ein Erkennungselement (122), das auf einer Außenseite der Nadel (30) vorgesehen ist; und
einen Erkennungssensor (124), um das Erkennungselement (122) zu erkennen, um die Messeinheit (40,70,80) anzutreiben.

12. Behandlungsvorrichtung nach Anspruch 11, wobei das Erkennungselement (122) auf der Außenseite der Nadel (30) in der Form eines Bands angeordnet ist.

13. Behandlungsvorrichtung nach Anspruch 11, wobei der Erkennungssensor (124) das Erkennungselement (122) durch Transmission und Empfang von Licht erkennt.

## Revendications

1. Dispositif de traitement comportant:
une sonde (10) pour acquérir une image d'une cible;
un guide d'aiguille (20) disposé le long de la sonde (10);
une aiguille (30) mobile le long du guide d'aiguille (20);
**caractérisé en ce que** le dispositif de traitement comporte en outre:
une unité de détection d'aiguille (120) pour déterminer si l'aiguille (30) a atteint une position de référence;
une unité de mesure (40, 70, 80) entraînée sur la base d'une détermination de l'unité de détection d'aiguille (120), l'unité de mesure (40, 70, 80) étant agencée pour détecter une distance de mouvement de l'aiguille (30) par rapport à la position de référence, basé sur le contact entre l'unité de mesure (40, 70, 80) et l'aiguille (30) se déplaçant le long du guide d'aiguille (20); et un contrôleur (50) pour calculer la distance du mouvement de l'aiguille (30) sur la base d'une valeur mesurée par l'unité de mesure (40, 70, 80).

2. Dispositif de traitement selon la revendication 1, dans lequel l'unité de mesure (40) comporte:
un pignon linéaire (42) disposé le long de l'aiguille (30);
un pignon circulaire (44) agencé pour tourner en prise avec le pignon linéaire (42); et
un organe de mesure de rotation (46) pour mesurer la rotation du pignon circulaire (44).

3. Dispositif de traitement selon la revendication 1, dans lequel l'unité de mesure (70) comporte:
un galet (72) agencé pour tourner en contact avec l'aiguille (30); et
un organe de mesure de rotation (46) pour mesurer la rotation du galet (72).

4. Dispositif de traitement selon les revendications 2 ou 3, dans lequel l'organe de mesure de rotation (46) est disposé sur la sonde (10) ou sur le guide d'aiguille (20).

5. Dispositif de traitement selon les revendications 2 ou 3, dans lequel l'organe de mesure de rotation (46) est disposé au milieu ou à une entrée du guide d'aiguille (20).

6. Dispositif de traitement selon la revendication 3, dans lequel l'unité de mesure (70) comporte en outre un organe de pression élastique (74) pour presser élastiquement l'aiguille (30) contre le galet (72).

7. Dispositif de traitement selon la revendication 1, dans lequel l'unité de mesure (80) comporte:
une protubérance de pression (82) ménagée sur l'aiguille (30) de façon protubérante;
et
un capteur de pression (84) en contact avec la protubérance de pression (82) pour mesurer la position de la protubérance de pression (82).

8. Dispositif de traitement selon la revendication 7, dans lequel la protubérance de pression (82) est formée sur une circonférence de l'aiguille (30).

9. Dispositif de traitement selon la revendication 7, dans lequel le capteur de pression (84) est configuré sous forme cylindrique entourant l'extérieur de l'aiguille (30).

10. Dispositif de traitement selon la revendication 1, dans lequel le contrôleur (50) est disposé sur n'importe lequel ou plus des éléments sélectionnés comprenant la sonde (10), le guide d'aiguille (20) et l'unité principale (60).

11. Dispositif de traitement selon la revendication 1, dans lequel l'unité de détection d'aiguille (120) comporte:
un élément de reconnaissance (122) disposé sur l'extérieur de l'aiguille (30);
et
un capteur de reconnaissance (124) pour reconnaître l'élément de reconnaissance (122) pour entraîner l'unité de mesure (40, 70, 80).

12. Dispositif de traitement selon la revendication 11, dans lequel l'élément de reconnaissance (122) est disposé sur l'extérieur de l'aiguille (30) sous la forme d'une bande.

13. Dispositif de traitement selon la revendication 11, dans lequel le capteur de reconnaissance (124) reconnaît l'élément de reconnaissance (122) par la transmission et la réception de lumière.
